# FASCICULE DE BREVET EUROPEEN

(11) **EP 1 301 194 B1**
(45) Date de publication et mention de la délivrance du brevet: **31.03.2010**
(21) Numéro de dépôt: 01955433.6
(22) Date de dépôt: 17.07.2001
(51) Int. Cl.: A61K 36/28, A61K 36/45, A61K 36/54, A61P 35/00

(54) **ASSOCIATION D'HUILES ESSENTIELLES ET APPLICATIONS THERAPEUTIQUES**
ETHERISCHEN ÖLEN KOMBINATION UND THERAPEUTISCHE VERWENDUNGEN
ESSENTIAL OIL COMBINATION AND THERAPEUTIC USES THEREOF

(30) Priorité: 20.07.2000 FR 0009515
(43) Date de publication de la demande: 16.04.2003
(73) Titulaire: Giraud, Anne Marie Pierrette, 13100 Aix en Provence (FR)
(72) Inventeur: Giraud, Anne Marie Pierrette, 13100 Aix en Provence (FR)
(74) Mandataire: Domange, Maxime
(86) Numéro de dépôt international: PCT/FR2001/002325
(87) Numéro de publication internationale: WO 2002/007744

(56) Documents cités:
- FR-A- 2 709 964
- US-A- 5 785 972
- DATABASE BIOSIS [en ligne] BIOSCIENCES INFORMATION SERVICE, PHILADELPHIA, PA, US; 1993 BELLEAU FRANCINE ET AL: "Composition of the essential oil of Ledum groenlandicum." Database accession no. PREV199396033061 XP002168059 & PHYTOCHEMISTRY (OXFORD), vol. 33, no. 1, 1993, pages 117-121, ISSN: 0031-9422
- DATABASE BIOSIS [en ligne] BIOSCIENCES INFORMATION SERVICE, PHILADELPHIA, PA, US; 1992 DE MEDICI D ET AL: "Chemical analysis of essential oils of Malagasy medicinal plants by gas chromatography and NMR spectroscopy." Database accession no. PREV199395030999 XP002168060 & FLAVOUR AND FRAGRANCE JOURNAL, vol. 7, no. 5, 1992, pages 275-281, ISSN: 0882-5734
- SCHNAUBELT K.: "Medical Aromatherapy, Healing with Essential Oils" 1999, FROG, LTD. ISBN: 1-883319-69-2 * page 205 *

## Description

La présente invention concerne une association d'huiles essentielles de plantes et son application thérapeutique, notamment dans le cas de pathologies cancéreuses et précancéreuses.

Les propriétés thérapeutiques des plantes aromatiques sont connues depuis l'Antiquité, que ce soit en Chine, en Egypte, en Grèce ou en Perse. Les Perses, mille ans avant notre ère, semblent être les inventeurs de la distillation proprement dite.
Toutefois, l'extraction des huiles essentielles (ci-après abrégée HE) par distillation à la vapeur d'eau ne s'est développée qu'au début du XIXe siècle en vue de l'industrialisation des produits agro-alimentaires de parfum.

Au début du XXe siècle des chercheurs comme Chamberland, Cadeac, Martindale démontrent par leur expérimentation le pouvoir antiseptique des huiles essentielles. Mais, l'aromathérapie moderne est née avec Gattefosse (1930) qui fut le premier à montrer les relations structure/activité des composants aromatiques et à codifier les grandes propriétés des arômes naturels : antitoxique, antiseptique, tonifiante, stimulante, calmante. L'aromathérapie n'est aujourd'hui encore que très peu utilisée par la médecine moderne. Une mauvaise utilisation des huiles essentielles suite à une méconnaissance des différentes variétés pour une même espèce sont vraisemblablement les raisons du manque de résultats observés cliniquement à ce jour. En effet, les huiles essentielles sont des corps complexes comprenant une multitude de molécules chimiques diverses ayant chacune des propriétés particulières liées vraisemblablement entre autres aux interactions physico-chimiques inter et intramoléculaires dans ces complexes.

Le développement de l'aromathérapie scientifique s'est accru avec les travaux de P. Franchomme qui a apporté la notion de "chémotype" ou race chimique définie des huiles essentielles. Chaque espèce ou chémotype évoluant dans son biotope produit une huile essentielle dont la structure moléculaire lui est propre car elle dépend de la nature du sol, de l'altitude, de l'ensoleillement et même des plantes avoisinantes.
On peut en précisant la famille, le genre, l'espèce et même la sous-espèce définir une race très différente d'une autre.

Ainsi trois critères sont indispensables pour définir une huile essentielle de qualité irréprochable :
- La plante doit être une espèce botanique certifiée et de race chimique définie (chémotypée).
- L'extraction doit se faire par distillation à la vapeur d'eau sous basse pression et sans détartrant chimique avec une eau pure et en respectant la durée de distillation de manière à inclure la fraction de tête composée des molécules aromatiques très volatiles et la fraction de queue riche en molécules plus lourdes moins volatiles entraînées après de nombreuses heures de distillation.
- L'huile essentielle (HE) doit être 100 % naturelle, c'est-à-dire non dénaturée par des molécules de synthèse chimique et 100 % pure, c'est-à-dire exempte d'autres HE proches.

Chaque huile essentielle est caractérisée par un certain nombre de constantes physiques permettant de l'identifier et de contrôler son origine géographique et sa pureté : densité, solubilité dans l'alcool, point de fusion et d'ébullition, point de congélation, pouvoir rotatoire (le pouvoir rotatoire étant tout particulièrement caractéristique des molécules naturelles), indice de réfraction. La comparaison des profils chromatographiques par chromatographie en phase gazeuse, la spectrométrie de masse et la résonance magnétique nucléaire (RMN) permet de déterminer les molécules chimiques composant l'HE.

D'une manière générale, la grande diversité des molécules chimiques contenues dans ces HE se traduit par des indications thérapeutiques très diverses.

L'Hélichrysum Italicum appelée Hélichryse italienne ou Immortelle appartient à la famille des Astéracées. Dans le genre Hélichryse on compte plus de 400 espèces. L'Hélichrysum Italicum se retrouve sur tout le pourtour de la Méditerranée, la sous-espèce (ssp) Serotinum vient de Corse et de Sardaigne. L'Hélichrysum Italicum sous-espèce Serotinum est connue pour ses propriétés antihématome exceptionnelles et antispasmodiques. Ses indications thérapeutiques sont donc essentiellement pour des affections circulatoires telles que, hématome, phlébites, érythroses.

Le Ledum Groenlandicum encore appelé Lédon du Groenland ou Thé du Labrador ou encore Bois de Savane appartient à la famille des Ericacées. On le trouve dans la province du Québec au nord du Canada. Cette plante pousse dans les marécages froids et les bois montagneux. Les propriétés thérapeutiques connues de cette HE du Ledon sont essentiellement les propriétés anti-inflammatoires, et antispasmodiques. Elle est également recommandée pour les indications hépatiques, telles que, intoxications hépatiques, insuffisances hépatiques ou séquelles d'hépatites virales.

La plante Ravensara Aromatica appartient à la famille des Lauracées qui se trouve dans les zones tropicales d'Amérique et d'Asie, plus particulièrement à Madagascar, l'île de la Réunion et sur l'île Maurice. Les indications thérapeutiques les plus connues de cette HE de Ravensara Aromatica sont les infections des voies respiratoires : bronchite, rhinite, sinusite. On rapporte également une action bénéfique en cas d'herpès labial, zona, varicelle, hépatite et entérite virales, ainsi qu'une action neurologique et psychologique pour le traitement de l'insomnie, du stress et de l'anxiété.

Les propriétés cytotoxiques des lactones et cetones sesquiterpéniques présentent dans certaines HE ont été étudiées. A ce jour, aucun résultat thérapeutique significatif n'a été rapporté sur ces molécules ou sur des HE en contenant et les auteurs (P. Franchomme "L'aromathérapie exactement" édition Roger Jollois) estiment même, que le recours aux HE ne permet pas de viser la lyse d'une tumeur maligne.

FR 2 709 964 décrit une utilisation de l'huile essentielle de Ravensara aromatica comme agent pharmaceutique anti viral. US 5,785,972 décrit une utilisation de l'huile essentielle d'Helichrysum Italicum dans le traitement des plaies et des brûlures. Medical Aromatherapy, Healing with essential oils by Kurt Schnaubelt, 1999, page 205 décrit l'utilisation thérapeutique de l'huile essentielle de Ledum groenlandicum.

Toutefois, on a découvert de façon surprenante selon la présente invention que l'association de trois HE, à savoir, Helichrysum Italicum ssp Serotinum, Ledum Groenlandicum, Ravensara Aromatica leur confère une synergie d'action se traduisant par des propriétés originales et plus particulièrement une amélioration de l'état général de patients, notamment les patients atteints de pathologies cancéreuses, précancéreuses, voire même une action anticancéreuse , démontrée de façon remarquable pour certains patients.

Plus précisément, la présente invention a pour objet l'association comprenant les trois dites huiles essentielles des plantes Helichrysum Italicum ssp Serotinum, Ledum Groenlandicum et Ravensara Aromatica pour une utilisation simultanée, séparée ou étalée dans le temps, pour le traitement de pathologies cancéreuses et précancéreuses.

Lesdites HE peuvent donc être formulées séparément ou de préférence en mélange.

Plus précisément une association selon la présente invention peut donc se présenter sous la forme d'un kit comprenant les différentes dites huiles essentielles (HE) formulées séparément ou sous la forme d'une unique composition comprenant les différentes dites huiles essentielles (HE) mélangées. Lorsque lesdites huiles essentielles (HE) sont formulées séparément, elles doivent être administrées simultanément ou juste l'une après l'autre ou encore de manière étalée dans le temps, de préférence espacées de 4 à 6 heures dans une même journée, par exemple matin, midi et soir.

De préférence l'association consiste dans une composition comprenant un mélange des trois dites huiles essentielles.

Après avoir mélangé lesdites huiles essentielles (HE), de préférence par simple agitation lente durant 30 secondes pour obtenir une homogénéisation parfaite, on laisse avantageusement le mélange au repos, de préférence à l'abri de la lumière à température ambiante, pendant au moins trois jours, afin d'obtenir la stabilité de cette nouvelle composition d'HE avant son utilisation thérapeutique.

Dans un mode particulier de réalisation l'association ou la composition selon l'invention comprend au moins 10 % en poids, de préférence au moins 20 % en poids de chacune desdites huiles essentielles par rapport au poids total desdites huiles essentielles présentes dans l'association ou composition.

Plus particulièrement, les formes galéniques utilisables sont notamment les suivantes :
- Formes orales liquides et diluées : sous forme de mélange avec des huiles végétales ou une solution alcoolique avec de l'alcool à 90°.
- Formes orales solides : sous forme de gélule ou capsule molle ou encore de mélange de l'huile pure ou diluée avec un support solide neutre tel que miel, comprimé de charbon, de lactose ou à défaut un morceau de sucre.
- Formes rectales ou vaginales : sous forme de suppositoire ou ovule.
- Formes cutanée : sous forme d'huile essentielle pure ou diluée.

La présente invention a également pour objet l'application à titre de médicament d'une association selon l'invention.

Plus particulièrement, la présente invention fournit une association pharmaceutique contenant une quantité thérapeutiquement efficace desdites huiles essentielles (HE).

Dans un mode d'administration particulier ladite quantité thérapeutiquement efficace correspond à 1 à 12 gouttes de chaque HE, soit 3 à 36 gouttes, pour les trois HE.

Plus particulièrement, une quantité efficace comprend environ 100 à 125 mg à administrer deux fois par jour, correspondant donc à une dose journalière de 6 à 12 gouttes, de préférence 8 à 10 gouttes, soit environ 225 à 250 mg par jour.

Comme démontré dans les exemples de réalisation décrits ci-après, une association médicamenteuse selon l'invention permet d'améliorer l'état général du patient et la tolérance à la chimiothérapie dans le cas de pathologies cancéreuses, dans certains états précancéreux

L'amélioration de l'état général évaluée selon la classification de Karnofsky, se traduit notamment par une meilleure tolérance aux traitements chimiothérapiques, celle-ci étant également évaluée en fonction des nausées et vomissements selon la classification de l'échelle de toxicité NCl.

Un traitement basé sur l'utilisation d'une association selon la présente invention peut être complémentaire vis-à-vis de thérapeutiques classiques en visant à améliorer l'état général du patient, voire à résorber les effets secondaires d'un traitement thérapeutique principal, notamment à améliorer les constantes biologiques, et plus particulièrement encore d'une amélioration de la tolérance à la chimiothérapie, en particulier dans le cas de pathologies cancéreuses ou précancéreuses.

Une association d'huiles essentielles (HE) selon la présente invention peut également être utilisée à titre de traitement principal pour conférer une activité anticancéreuse comme il sera explicité dans les exemples ci-après.

L'intérêt de l'association d'huiles essentielles selon la présente invention prend également toute son importance dans le traitement des préventions des récidives de cancer.

Des travaux de recherche sont en cours pour élucider les mécanismes et les molécules qui participent aux actions thérapeutiques de la présente invention. L'activité thérapeutique semble s'appuyer sur deux mécanismes distincts mais complémentaires, à savoir : un mécanisme biochimique et un mécanisme énergétique. Cette synergie biochimique et énergétique semble indispensable pour que l'activité thérapeutique recherchée soit obtenue.

On utilise de préférence l'huile essentielle d'Helichrysum Italicum ssp Serotinum contenue dans la sommité fleurie extrait au moyen de la distillation par entraînement à la vapeur d'eau. Les rendements réels obtenus sont faibles, ils oscillent entre 0,9 et 1,1‰ pour une durée de distillation de 1h 45 à 2 heures suivant l'état de la dessiccation de la plante. 100 Kg de sommités fleuries d'Helichryse fourniront au maximum 110 ml d'HE. les données physiques et biochimiques de cette HE d'Helichryse sont les suivantes :

| | |
|---|---|
| ➢ Densité à 20° : | 0,910 |
| ➢ Indice de réfraction : | 1,473 à 1,476 |
| ➢ Pouvoir rotatoire : | -2°20' à +0°10' |
| ➢ Caractéristiques organoleptiques : | * fluide et mobile |
| | * du jaune clair à jaune verdâtre |
| | * odeur forte, enivrante, éthérée et caractéristique |

Les données fournies par la chromatographie en phase gazeuse couplées à la spectrométrie de masse de cette HE d'Helichryse permettent d'identifier les molécules principales suivantes regroupées par familles chimiques :
Esters :
   ➢ acétate de néryl
   ➢ propionate de néryl
   ➢ pentanoate de néryl
Monoterpènes :
   ➢ pinène alpha
   ➢ limonène
   ➢ pinène bêta
   ➢ terpinène gamma
   ➢ terpinolène
Sesguiterpène :
   ➢ curcumène gamma
Monoterpènols :
   ➢ nérol
   ➢ terpinène 1 ol 4
   ➢ terpinéol alpha
   ➢ linalol
Sesquiterpenols :
   ➢ guaiol
Diones :
   ➢ triméthyl - 2,5,7 dec- 2 en dione - 6,8
   ➢ dicétone bêta
Cétones :
   ➢ undécanone 2
   ➢ nonanone 2
Oxydes :
   ➢ cinéol 1,8
   ➢ oxyde de nérol
Aldéhyde :
   ➢ citronallal

On utilise de préférence l'huile essentielle de Ledum Groenlandicum extraite du rameau fleuri ou du bourgeon. Pour le rameau fleuri le rendement de la distillation varie de 0,16 à 0,61 % (masse d'huile obtenue par rapport à la masse végétale fraîche avant extraction). Le temps de distillation est relativement long, supérieur à 5 heures.

**Les données physiques et biochimiques sont les suivantes :**

| | |
|---|---|
| ➢ Densité : | 0,8493 à 0,8714 |
| ➢ Indice de réfraction : | 1,4614 à 1,5159 |
| ➢ Pouvoir rotatoire : | +2,6° |
| ➢ Caractéristiques organoleptiques : | * fluide et mobile |
| | * jaune pale |
| | * odeur basalmique avec une note herbacée |

Les données fournies par la chromatographie en phase gazeuse couplées à la spectrométrie de masse permettent d'identifier dans la composition chimique de l'huile essentielle du Ledum Groelendicum les molécules principales suivantes :
Terpènes :
   ➢ pinène alpha
   ➢ camphène
   ➢ pinène bêta
   ➢ sabinène
   ➢ terpinène alpha
   ➢ terpinène gamma
   ➢ limonène
   ➢ terpinolène
   ➢ phéllandrène bêta
   ➢ para cymène
Sesquiterpènes :
   ➢ caryophyllène bêta
   ➢ humulène alpha
   ➢ germacrène
   ➢ sélinène alpha
   ➢ sélinène bêta
   ➢ farnésène alpha
   ➢ élemène gamma
Alcools :
   ➢ nérol
   ➢ terpinéol alpha
   ➢ lédol
Aldéhyde :
   ➢ myrténal
Cétones :
   ➢ thujone bêta
   ➢ germacrone

S'agissant de l'huile essentielle de Ledum Groenlandicum on peut utiliser une variété d'HE obtenue à partir du bourgeon ou du rameau fleuri, la concentration en germacrone étant plus importante dans les extraits obtenus à partir du bourgeon.

On utilise de préférence l'huile essentielle de Ravensara Aromatica extraite des jeunes rameaux feuillus. Le rendement de distillation varie de 4 à 8 %, le temps de distillation s'échelonne entre 10 et 15 heures. 100 Kg de feuilles de Ravensara fournissent jusqu'à 800 ml d'HE.

**Les données physiques et biochimiques sont :**

| | |
|---|---|
| ➢ Densité à 20°: | 0,910 à 0,919 |
| ➢ Indice de réfraction : | 1,483 à 1,490 |
| ➢ Pouvoir rotatoire : | 1°18 à 1°38 |
| ➢ Caractéristiques organoleptiques : | * fluide et mobile |
| | * du jaune pâle à l'incolore |
| | * odeur caractéristique où la fraîcheur du 1,8 cinéole se mêle à la chaleur du terpinéol alpha |
| | * saveur tonique, dynamique et astringente |

Les données fournies par la chromatographie phase gazeuse couplées à la spectrométrie de masse de cette HE de Ravensara Aromatica permettent de déceler les molécules principales de l'HE suivantes :
Oxyde monoterpénique :
   ➢ cinéol 1,8
Ester :
   ➢ acétate de terpinyle alpha
Monoterpènes :
   ➢ sabinène
   ➢ pinène alpha
   ➢ pinène bêta
   ➢ camphène
   ➢ limonène
   ➢ phéllandrène alpha
   ➢ humuléne alpha
   ➢ thujène
   ➢ terpinène alpha
   ➢ terpinène gamma
Monoterpènols et alcools :
   ➢ terpinène 1 ol 4
   ➢ terpinéol alpha
Phénol :
   ➢ eugénol
Sesguiterpènes :
   ➢ caryophyllène bêta
   ➢ germacrène
Aldéhyde :
   ➢ myrténal
Ether :
   ➢ eugénol de méthyl

La posologie et le pourcentage des trois HE, Helichrysum Italicum, Ledum Groenlandicum et Ravensara Aromatica ainsi que la durée du traitement, peuvent varier et dépendre de plusieurs facteurs, tels que : le stade de la maladie, notamment du cancer, histologie du cancer, association ou non avec une radiothérapie, chimiothérapie, état général du patient. L'action thérapeutique de l'association selon l'invention est utile dans les différentes phases de la maladie, notamment du cancer en soins palliatifs, en soins curatifs ou en soins préventifs.

En soins palliatifs, on observe que l'action dynamisante du Ravensara améliore l'état clinique du patient avec une diminution de la fatigue, une atténuation des douleurs, une amélioration de l'appétit et du sommeil. La voie cutanée pour l'administration de l'association selon la présente invention peut être avantageuse à ce stade.

En soins curatifs, le traitement avec une association de trois HE selon la présente invention peut être donné pendant toute la période qui précède une intervention chirurgicale et poursuivi après jusqu'à l'attente des résultats et des traitements ultérieurs de chimiothérapie et/ou radiothérapie. L'action de l'association à base de trois HE selon la présente invention permet de :
- Limiter l'évolution de la tumeur (action anticancéreuse) ;
- Diminuer les saignements pendant l'intervention, saignements qui sont parfois responsables de métastases par voie sanguine, cette très belle action antihémorragique particulièrement liée à l'Helichryse italienne ;
- Préparer le patient à mieux supporter l'anesthésie due à l'action particulière du Ledum Groenlandicum qui désintoxique l'organisme par sa puissante action sur le foie qui permet de mieux éliminer les produits de l'anesthésie, les patients constatant effectivement un très bon réveil à l'anesthésie ;
- Améliorer la cicatrisation par l'action de l'Helichryse ;
- Améliorer l'état psychologique du patient qui est souvent très inquiet, angoissé, stressé, insomniaque, découragé quand on lui annonce une forte probabilité de cancer.

Un traitement combiné avec une chimiothérapie présente un triple intérêt, à savoir :
- Une activité anticancéreuse de l'association elle-même ;
- Une meilleure tolérance de la chimiothérapie, que ce soit au niveau biologique avec une normalisation du bilan hépatique et rénal ou au niveau clinique avec une diminution de l'asthénie et des nausées, vomissements ;
- Une stimulation immunitaire vraisemblablement plus particulièrement liée au Ravensara.

Pour un traitement avec une association selon la présente invention en complément d'un traitement de chimiothérapie, la durée du traitement doit être aussi longue que la durée de la chimiothérapie (en moyenne six mois avec des arrêts de une à deux semaines tous les mois ou tous les deux mois). La posologie et les pourcentages des trois huiles essentielles seront variables en fonction du patient et du stade de la maladie et des produits utilisés pour la chimiothérapie.

En soins préventifs, une association de trois HE selon la présente invention peut être utilisée en moyenne quatre à huit semaines deux fois par an, au printemps et à l'automne ou trois à quatre fois par an en fonction du stade du cancer. En moyenne ce traitement préventif durera de cinq à dix ans.

Une association préférée selon la présente invention comprend les composés suivants :
- le cinéol 1,8 ;
- au moins un alcool choisi parmi le terpinéol alpha et le terpinène 1 ol 4 ;
- des composés terpéniques choisis parmi les composés comprenant le sabinène, le pinène, le terpinène, le limonène, le phéllandrène, le terpinolène, le sélinène, le curcumène, le germacrène, le cymène, le caryophyllène, le thujène et l'ocimène ;
- des esters choisis parmi l'acétate de néryl et le propionate de néryl, et
- des composés cétoniques comprenant au moins une cétone sesquiterpénique, de préférence la germacrone et au moins une autre cétone choisie de préférence parmi la nonanone, la carvone, l'undécanone et la triméthyl -2,5,7 dec-2 en dione-6,8.

Plus particulièrement, une association selon la présente invention comprenant un mélange de trois huiles essentielles comprend les composés principaux suivants :
- le cinéol 1,8 ;
- le terpinéol alpha ;
- l'acétate de néryl ;
- la germacrone, et
- des composés terpènes choisis parmi le germacrène, le sabinène, le limonène, le phéllandrène, et le sélinène.

Plus particulièrement encore, une association selon l'invention comprend les composés suivants :
- le cinéol 1,8 ;
- le terpinène 1 ol 4 ;
- le terpinéol alpha ;
- le dicétone bêta ;
- l'acétate de néryl ;
- la germacrone ;
- le limonène et
- le germacrène.

D'autres caractéristiques, avantages et résultats de la présente invention apparaîtront à la lumière des exemples détaillés de réalisation qui suivent.

### A - ASSOCIATIONS D'HUILES ESSENTIELLES

Dans les exemples de réalisation ci-après, on a utilisé des associations des HE de Helichrysum Italicum ssp Serotinum, Ledum Groelandicum et Ravensara Aromatica préférées telles que caractérisées précédemment. L'huile essentielle de Ledum Groelandicum était extraite à partir du rameau fleuri.

On a mélangé les huiles essentielles selon le procédé suivant : simple agitation lente du mélange durant 30 secondes, laissé à l'abri de la lumière dans un verre teinté à température ambiante pendant trois jours.

Dans le cas d'une composition comprenant les trois HE en mélange dans les proportions en poids suivantes :
- 1/3 d'Helichrysum Italicum ssp Serotinum,
- 1/3 de Ravensara Aromatica,
- 1/3 de Ledum Groenlandicum.

**Les données fournies par une chromatographie en phase gazeuse couplée à la spectrométrie de masse, la composition chimique du mélange était la suivante :**

| | |
|---|---|
| ➢ tricylène | 0,01 % |
| ➢ thujène alpha | 0,29 % |
| ➢ pinène alpha | 2,34 % |
| ➢ fenchène alpha | 0,18 % |
| ➢ camphène | 0,27 % |
| ➢ isopropyl méthyl benzène | 0,02 % |
| ➢ sabinène | 4,72 % |
| ➢ pinène bêta | 1,62 % |
| ➢ myrcène | 0,58 % |
| ➢ phéllandrène alpha | 0,42 % |
| ➢ carène delta | 0,29 % |
| ➢ para cymène | 1,57 % |
| ➢ limonène | 14,11 % |
| ➢ eucalyptol | 12,7 % |
| ➢ phéllandrène bêta | 6,95 % |
| ➢ ocimène trans bêta | 0,16 % |
| ➢ terpinène gamma | 0,7 % |
| ➢ trans thujanol 4 | 0,15 % |
| ➢ diméthyl-2,4 dione-3,5 heptane | |
| ➢ nonanone 2 | 0,02 % |
| ➢ para diméthyl styrène | 0,43 % |
| ➢ terpinolène | 0,2 % |
| ➢ linalol | 0,5 % |
| ➢ cis thujanol 4 | 0,08 % |
| ➢ alcool fenchyl | 0,02 % |
| ➢ cis para 2-8 menthadiène en 1 ol | 0,25 % |
| ➢ isoamyl angelate | 0,01 % |
| ➢ trans pino carvéol | 0,2 % |
| ➢ bornéol | 0,19 % |
| ➢ terpinène 1 ol 4 | 1 ,26 % |
| ➢ cis para mentha 1-(7),8 diène-2 ol | 2,64 % |
| ➢ terpinéol alpha | 3,11 % |
| ➢ transdihydro carvone carvéol | 0,29 % |
| ➢ trans carvéol | 0,1 % |
| ➢ trans para mentha 1-(7),8 diène 2 ol | 1,6 % |
| ➢ nérol | 1,05 % |
| ➢ cis carveol | 0,2 % |
| ➢ carvone | 0,71 % |
| ➢ cuminaldéhyde | 0,01 % |
| ➢ perrilaldéhyde | 0,02 % |
| ➢ acétate de bornyle | 0,42 % |
| ➢ undécanone 2 | 0,02 % |
| ➢ élemène delta | 0,01 % |
| ➢ acétate de néryl | 14,98 % |
| ➢ acétate de géranyle | 0,13 % |
| ➢ isoitalecène | 0,04 % |
| ➢ copaène alpha | 0,21 % |
| ➢ élemène bêta | 0,35 % |
| ➢ itacélène | 0,3 % |
| ➢ cis bergamotène alpha | 0,1 % |
| ➢ gurjunène alpha | 0,64 % |
| ➢ 25,7-triméthyl dec-2 en 6,8 dione | 0,8 % |
| ➢ trans caryophyllène bêta | 0,52 % |
| ➢ néryl propionate | 2,03 % |
| ➢ cis farnesène bêta | 0,12 % |
| ➢ humulène alpha | 1,14 % |
| ➢ acoradiène alpha | 0,18 % |
| ➢ allo aromadendrène | 0,36 % |
| ➢ gurjunème gamme | 0,33 % |
| ➢ curcumène alpha | 1,37% |
| ➢ curcumène gamma | 3,27 % |
| ➢ sélinène bêta | 4,03 % |
| ➢ sélinène alpha | 0,85 % |
| ➢ cis bisabolène alpha | 1,41 % |
| ➢ bicyclogermacrène | 0,37 % |
| ➢ curcumène bêta | 0,95 % |
| ➢ 7 épi sélinène alpha | 0,02 % |
| ➢ cadinène delta | 0,21 % |
| ➢ butyrate de géranyl | 0,02 % |
| ➢ trans bisabolène alpha | 0,02 % |
| ➢ trans nérolidol | 0,02 % |
| ➢ germacrène B | 0,42 % |
| ➢ 2,5,7 triméthyl dec 2 en 6,8 dione | 0,02 % |
| ➢ palustrol | 0,25 % |
| ➢ isovalérate de néryl | 0,25 % |
| ➢ guaiol | 0,47 % |
| ➢ germacrone | 1,54 % |
| ➢ tiglate de géranyl | 0,01 % |
| ➢ ledol | 0,06 % |
| ➢ eudesmol gamma | 0,56 % |
| ➢ eudesmol bêta | 0,34 % |
| ➢ eudesmol alpha | 0,36 % |
| ➢ bulnésol | 0.2% |
| TOTAL | 99,92 % |

Dans cette liste les produits les plus caractéristiques sont les suivants :
- L'eucalyptol (12,7 %) qui correspond au cinéol 1,8 et appartient à la famille des oxydes ;
- Le terpinéol alpha (3,11 %) qui appartient à la famille des alcools ;
- La germacrone (1,54 %) qui appartient à la famille des cétones sesquiterpéniques ;
- D'autres composés cétoniques (1,55 %) comprenant la nonanone (0,02 %), carvone (0,71 %), undécanone (0,02 %) et 2,5,7 triméthyl dec-2 en 6,8 dione (0,8 %) ;
- 51,85 % de molécules terpéniques, à savoir le tricyclène (0,01 %), le thujène alpha (0,29 %), le pinène alpha (2,34 %), le fenchène alpha (0,18 %), le camphène (0,18 %), le sabinène (4,72 %), le pinène bêta (1,62 %), le myrcène (0,58 %), le phéllandrène alpha (0,42 %), le carène delta (0,29 %), le para cymène (1,57 %), le limonène (14,11 %), le phéllandrène (6,95 %), le trans ocimène bêta (0,16 %), le terpinène gamma (0,7 %), le para diméthyl styrène ((0,43), le terpinolène (0,2 %), l'élemène delta (0,01 %), l'isoitalecène (0,04 %), le copaène alpha (0,21 %), l'élemène bêta (0,35 %), l'italecène (0,3 %), le cis bergamotène alpha (0,1 %), le gurjunème alpha (0,64 %), trans caryophyllène bêta (0,52 %), le cis farnesène bêta (0,12 %), l'humulène alpha (1,14 %), l'acoradiène alpha (0,18 %), l'allo aromadendrène (0,36 %), le gurjunème gamma (0,33 %), le curcumène alpha (1,37 %), le curcumène gamma (3,27 %), le sélinène bêta (4,03 %), le sélinène alpha (0,85 %), le bisabolène alpha (0,02 %) et le germacrène (0,42 %) ;
- Les esters comprenant l'acétate de néryl (14,98 %) et le néryl de propionate (2,03 %).

### B - APPLICATIONS THERAPEUTIQUES

240 patients ont été traités par une composition d'HE comprenant au moins deux des trois HE décrites ici. 220 patients étaient atteints d'une pathologie cancéreuse et 20 patients atteints d'hépatites B ou C ou B et C aiguës ou chroniques.

Les types de cancers comprenaient principalement des cancers de sein (110 cas), ovariens (14 cas), du colon (24 cas), de la prostate (10 cas) et pulmonaire (14 cas).

### B.I. - Traitement de pathologies cancéreuses

### 1 - Traitement curatif

163 patients ont bénéficié du traitement d'association d'HE décrites ici dans le cadre d'un traitement curatif.

On a constaté une très nette amélioration de la tolérance à la chimiothérapie avec l'utilisation de l'association d'HE avec une amélioration de l'état général d'après la classification de Karnofsky dans 90 % des cas, les nausées et vomissements étant en nette diminution dans le cas de traitement avec une association d'HE selon l'invention.

1.1. - Pour tous les patients traités en complément et conjointement à une chimiothérapie on a observé :
- une amélioration de la tolérance au traitement,
- une meilleure récupération après les traitements,
- une activité antalgique remarquable, et
- une meilleure immunité.

1.2. - Sur les 10 patients ayant pu bénéficier d'un traitement avant intervention chirurgicale Il a pu être constaté les faits suivants :
- arrivée à l'intervention dans un état très calme et sans angoisse,
- réveil à l'anesthésie excellent,
- récupération après l'intervention très rapide et d'un bon tonus,
- cicatrisation parfaite.

En particulier, ces patients ont eu un très bon réveil à l'anesthésie, avec très peu de saignement, le redon étant enlevé en moyenne au deuxième jour, alors que la moyenne habituelle d'ablation se situe entre le troisième et le quatrième jour. Et, on a pu constater une cicatrisation beaucoup plus rapide et excellente. Aucun épanchement lymphatique n'a été constaté dans les suites opératoires.

1.3. - Certains cas ont montré une activité cytotoxique en l'absence de traitement complémentaire de chimiothérapie.

Les observations suivantes montrant une action cytoxique des associations d'HE selon l'invention par action de lyse de la cellule tumorale sur des adénocarcinomes sont fournis à titre illustratif.

a) Une patiente âgée de 41 ans a subi une intervention pour un adénocarcinome du sein gauche. Des résultats anatomo-pathologiques ont révélé une lésion contenant des cellules cancéreuses. Il a donc été prévu une nouvelle intervention chirurgicale. La deuxième intervention a eu lieu six semaines après. Entre les deux interventions l'unique traitement pris par la patiente a été une association à raison de 4 gouttes matin et soir pendant un mois dans les proportions pondérales suivantes :
- Helichrysum Italicum 40 %,
- Ledum Groenlandicum 50 %
- Ravensara Aromatica 10 %.

Après la première intervention, la personne présentait un hématome important au niveau du sein. Après trois jours de traitement avec l'association des HE selon l'invention, l'hématome avait disparu. Les résultats anatomo-pathologiques après la deuxième intervention n'ont mis en évidence aucune lésion carcinomateuse.

b) Une patiente âgée de 76 ans a subi une mammographie et échographie mammaire de dépistage. Cette échographie a révélé dans le sein gauche la présence d'une lacune solide atténuant la conduction ultrasonore de 13 mm et la mammographie du sein gauche montrait une opacité aux contours spiculés associée à des microcalcifcations. Cette image visualisée en échographie et à la mammographie permettait de diagnostiquer un cancer du sein et nécessitait un contrôle histologique. La patiente refusant tout traitement chimiothérapique ou radiothérapique n'a pris que comme seul traitement médical une association d'HE à raison de 6 gouttes matin et soir pendant deux mois comprenant la composition pondérale suivante :
- Ledum Groenlandicum à 50 %,
- Helichrysum Italicum 30 %
- Ravensara Aromatica 20 % ;

A l'issu du traitement à la mammographie on a noté une très nette atténuation de la densité hétérogène. L'échographie ne montrait plus la présence d'une lésion évolutive. La disparition totale de la lacune solide visualisée deux mois auparavant et la régénération de tissus sains a révélé un effet cytoxique du complexe d'HE administré.

c) Une patiente âgée de 58 ans a été opérée d'un cancer du sein gauche une mastectomie avec curage axillaire ayant été réalisée. La patiente avait par ailleurs été traitée par radiothérapie et une anti-hormonothérapie avec du Nolvadex® à raison de 20 mg par jour pendant cinq ans. Sept ans après l'opération la patiente présentait des douleurs vertébrales qui étaient en rapport avec des métastases osseuses confirmées par scintigraphie ; et une élévation des marqueurs CA15-3 à 700 UI/l. La patiente a alors suivi une radiothérapie à visée antalgique et un traitement complémentaire par Femara®. Une chimiothérapie devait être commencée, mais celle-ci fut refusée par la patiente. Un traitement par HE a été proposé à raison de 5 gouttes matin et soir avec la composition pondérale suivante :
- Helichrysum Italicum 30 %,
- Ledum Groenlandicum 50 %,
- Ravensara Aromatica 20 %.

Ce traitement a été poursuivi pendant plusieurs mois. Au fil des mois, l'état général de la patiente a été très amélioré au niveau des douleurs, sa marche est redevenue normale alors qu'elle se déplaçait avec beaucoup de difficulté. On a pu constater une diminution progressive des marqueurs tumoraux avec normalisation. Une nette régression des métastases osseuses a été confirmée par scintigraphie. Il a été estimé que le traitement complémentaire à base d'association d'HE selon l'invention avait joué un rôle déterminant dans la régression des lésions, compte tenu que le seul traitement par le Femara® n'aurait pas permis une telle amélioration.

d) Une patiente âgée de 54 ans présentait une tumeur du colon (adénocarcinome bien différencié). La patiente a suivi pendant 10 jours avant son intervention chirurgicale, à raison de 12 gouttes 3 fois par jour, l'association avec la composition pondérale suivante :
- Helichrysum Italicum 25 %,
- Ledum Groenlandicum 25 %,
- Ravensara Aromatica 50 %.

En dix jours de traitement, on a pu constater une nette régression tumorale. La patiente a poursuivi son traitement d'huiles essentielles pendant 3 mois, ne bénéficiant d'aucun traitement complémentaire.

e) Un patient âgé de 63 ans présentait une néoplasie du rectum avec métastases depuis 1992. Ce patient a subi de multiples protocoles de chimiothérapie sans résultat, ainsi qu'une gastrectomie pour métastases gastriques de son cancer rectal en 1999. On a constaté une aggravation des métastases et une augmentation du taux d'ACE (marqueur de l'activité néoplasique). Devant l'aggravation des métastases et l'augmentation progressive du taux d'ACE, l'arrêt de la chimiothérapie a été décidé. Le patient a commencé un traitement d'huiles essentielles avec les compositions pondérales suivantes, à raison de 12 gouttes 3 fois par jour :
- Helichrysum Italicum 25 %,
- Ledum Groenlandicum 25 %,
- Ravensara Aromatica 50 %.

Un mois après le début du traitement d'huiles essentielles, on a constaté une diminution franche du taux d'ACE de 202 à 133, puis 10 jours après à 111.

Il est à noter que le patient a pris ses huiles essentielles en absorption sublinguale et non digestive, compte tenu de l'ablation de l'estomac. Le patient conservait en sublingual l'association d'huiles essentielles pendant une durée de 20 à 30 mn en début de traitement puis seulement 1 à 2 mn un mois plus tard, compte tenu de l'irritation de la muqueuse buccale. Le patient présentait un excellent état général.
La voie d'absorption sublinguale semble déterminante dans l'activité cytotoxique et antalgique.

### 2 - Traitements préventifs

31 patients ont bénéficié du traitement d'une association de trois HE selon la présente invention dans le cadre d'un traitement préventif des récidives.

Parmi ces cas :
- certains n'avaient aucune symptomatologie particulière avant le début du traitement.
- D'autres patients présentaient une symptomatologie :
   - Certains patients présentaient une altération de leur état général, avec un score de Karnofsky à 80 avant début de traitement. Après 2 semaines de traitement le score était passé à 100.
   - D'autres patients présentaient des troubles digestifs (ballonnements, constipation). 3 patients sur 5 ont été améliorés à 15 jours de traitement en moyenne.

A ce jour, le recul n'est pas suffisant pour pouvoir interpréter les résultats.
Toutefois, d'une façon générale, on peut constater que les patients pratiquant leur cure deux à trois fois par an constatent une amélioration de leur état général et de leur immunité.

### 3 - Soins palliatifs

26 cas de patients ont bénéficié d'un traitement d'une association de Ledum Groenlandicum, Helichrysum Italicum ssp Serotinum et Ravensara Aromatica au stade terminal de cancer de cancer pulmonaire, de cancer à l'estomac et de cancer au colon, dans le cadre de soins palliatifs.

Les patients sont décédés mais le confort de vie a été amélioré dès l'utilisation de l'association des trois HE. L'action tonifiante et stimulante de l'HE Ravensara Aromatica a pu être constatée.

On a pu constater une atténuation des douleurs avec possibilité de diminuer les antalgiques, et dans certains cas une augmentation de la survie de façon significative.

### Exemple 1 : Formulations de compositions pour traitement de cancer

### 1) Soins palliatifs

On utilisera la formulation d'HE selon la proportion pondérale suivante :
HE Ravensara Aromatica 40 %,
HE Ledum Groenlandicum 30 %,
HE Helichrysum Italicum ssp Serotinum 30 %.

Posologie : 8 à 10 gouttes par jour en deux prises.

Durée du traitement : 3 mois en continu puis arrêt de 10 jours.

Si amélioration : reprise du traitement ci-dessus, 3 semaines par mois pendant 3 mois, puis 10 jours par mois pendant 6 mois.

En l'absence d'amélioration : arrêt du traitement pendant un mois puis en fonction de l'état du patient nouveau traitement avec la formule de proportions pondérales suivantes :
HE Ravensara Aromatica 10 %,
HE Ledum Groenlandicum 45 %,
HE Helichrysum Italicum ssp Serotinum 45 %.

Posologie : 6 à 8 gouttes par jour en deux prises pendant un mois, puis 15 jours par mois.

### 2) Pour des soins curatifs

### 2.1 Avec chimiothérapie

Formulation selon les proportions pondérales suivantes :
HE Ravensara Aromatica 30 %,
HE Ledum Groenlandicum 40 %,
HE Helichrysum Italicum ssp Serotinum 30 %.

Posologie : 6 à 8 gouttes par jour en deux prises.

Durée du traitement : pendant toute la période de chimiothérapie, soit 5 jours sur 7, soit 3 semaines par mois.

### 2.2 Sans chimiothérapie

- Cancer aux stades I et II

Formulation selon les proportions pondérales suivantes :
HE Ravensara Aromatica 30 %,
HE Ledum Groenlandicum 40 %,
HE Helichrysum Italicum ssp Serotinum 30 %.

Posologie : 6 gouttes par jour en deux prises.

Durée du traitement : trois mois avec une semaine d'arrêt par mois.
- Cancer aux stades plus évolués III - IV

Formulation selon les proportions pondérales suivantes :
HE Ravensara Aromatica 30 %,
HE Ledum Groenlandicum 60 %,
HE Helichrysum Italicum ssp Serotinum 10 %.

Posologie : 6 gouttes par jour pendant 3 à 6 mois avec arrêt une semaine par mois.
- Avant intervention chirurgicale :

Formulation selon les proportions pondérales suivantes :
HE Ravensara Aromatica 30 %,
HE Ledum Groenlandicum 40 %,
HE Helichrysum Italicum ssp Serotinum 30 %.

Posologie : 4 gouttes par jour pendant toute la durée qui précède l'intervention et poursuivie après jusqu'à l'attente des résultats.

### 3) Prévention des récidives

### 3.1 Stades I et II

Formulation selon les proportions pondérales suivantes :
HE Ravensara Aromatica 20 %,
HE Ledum Groenlandicum 40 %,
HE Helichrysum Italicum ssp Serotinum 40 %.

Posologie : 6 gouttes par jour en deux prises.

Durée du traitement : six semaines 3 ou 4 fois par an.

### 3.2 Stades III et IV

Formulation selon les proportions pondérales suivantes :
HE Ravensara Aromatica 30 %,
HE Ledum Groenlandicum 50 %,
HE Helichrysum Italicum ssp Serotinum 20 %.

Posologie : 6 gouttes par jour en deux prises.

Durée du traitement : 6 semaines deux fois par an.

La durée totale du traitement préventif est valable en moyenne 5 à 10 ans.

### Exemple 2 : Formulations des compositions pour pathologies précancéreuses

Une association de trois HE selon l'invention est intéressante pour traiter les états dysplasiques en rapport avec des virus, en particulier une dysplasie du col et une papillomatose laryngée due papilloma virus est intéressante de par l'action antivirale puissante et stimulante due aux HE Ravensara Atomatica associée à l'action de lyse des cellules dysplasiques par l'association de Ledum Groenlandicum et Helichrysum Italicum ssp Serotinum.

### 2.1. - formulation pour dysplasie du col (due aux papillomavirus) (proportions pondérales) :

HE Ravensara Aromatica 40 %,
HE Ledum Groenlandicum 40 %,
HE Helichrysum Italicum ssp Serotinum 20 %.

Posologie : 5 à 6 gouttes matin et soir.

Durée du traitement : 3 semaines puis arrêt d'1 semaine puis reprise 3 semaines.

Pour un traitement local sous forme d'ovules, la formulation suivante peut être proposée :
HE Ravensara Aromatica 100 mg
HE Ledum Groenlandicum 50 à 100 mg selon le stade de dysplasie (dysplasie légère 50 mg, dysplasie moyenne 75 mg, dysplasie sévère 100 mg),
HE Helichrysum Italicum ssp Serotinum 50 mg.

### 2.2. - Formulation pour Condylome simple (sans dysplasie) du col utérin (proportion pondérale)

HE Ravensara Aromatica 150 mg,
HE Helichrysum Italicum ssp Serotinum 100mg,
HV Colophyllum Inophyllum 50 mg

Posologie : 1 ovule matin et soir la première semaine. Puis 1 ovule par jour les 2^{ème} et 3^{ème} semaines avec arrêt 8 jours avant une deuxième cure identique à la première.

Après un contrôle colposcopique poursuivre le traitement encore 2 mois en cas de disparition seulement partielle.

### 2.3. - Formulation pour polypes de colon et vessie (proportions pondérales) :

HE Ravensara Aromatica 20 %,
HE Ledum Groenlandicum 40 %,
HE Helichrysum Italicum ssp Serotinum 40 %.

Posologie : 6 gouttes matin et soir per os.

Durée du traitement : 6 semaines.

Fréquence du traitement : tous les 6 mois, soit deux fois par an (printemps et automne pendant 10 à 20 ans).

## Revendications

1. Association comprenant trois huiles essentielles consistant dans les huiles essentielles de Helichrysum Italicum ssp Serotinum, Ledum Groenlandicum et Ravensara Aromatica, pour une utilisation simultanée, séparée ou étalée dans le temps, pour le traitement de pathologies cancéreuses et précancéreuses.

2. Association pour une utilisation selon la revendication 1 **caractérisée en ce qu'**elle consiste dans une composition comprenant en mélange les trois dites huiles essentielles de Helichrysum Italicum ssp Serotinum, Ledum Groenlandicum et Ravensara Aromatica.

3. Association pour une utilisation selon l'une des revendications 1 ou 2 **caractérisée en ce qu'**elle comprend au moins 10 % en poids, de préférence au moins 20 % en poids de chacune desdites huiles essentielles par rapport au poids total desdites huiles essentielles.

4. Association pharmaceutique pour une utilisation selon l'une des revendications 1 à 3 **caractérisée en ce qu'**elle comprend les composés suivants :
- le cinéol 1,8 ;
- au moins un alcool choisi parmi le terpinéol alpha et le terpinène 1 ol 4 ;
- des composés terpéniques choisis parmi les composés comprenant le sabinène, le pinène, le terpinène, le limonène, le phéllandrène, le terpinolène, le sélinène, le curcumène, le germacrène, le cymène, le caryophyllène, le thujène, l'ocimène ;
- les esters comprenant l'acétate de néryl et/ou le propionate de néryl, et
- les composés cétoniques comprenant au moins une cétone sesquiterpénique, de préférence la germacrone, et au moins une cétone monoterpénique choisie parmi la nonanone, carvone, undécanone, et triméthyl- 2,5,7 dec-2 en dione-6,8.

5. Association pour une utilisation selon la revendication 4 **caractérisée en ce qu'**elle comprend les composés principaux suivants :
- le cinéol 1,8 ;
- le terpinéol alpha;
- l'acétate de néryl ;
- la germacrone, et
- des composés terpènes choisis parmi le germacrène, le sabinène, le limonène, le phéllandrène, et le sélinène.

6. Association pharmaceutique pour une utilisation selon l'une des revendications 1 à 5, **caractérisée en ce qu'**elle vise à traiter une tumeur.

7. Association pharmaceutique pour une utilisation selon l'une des revendications 1 à 5, **caractérisée en ce qu'**elle vise à améliorer l'état général du patient et la tolérance à la chimiothérapie.

## Claims

1. Association comprising three essential oils consisting in the essential oils of *Helichrysum italicum* ssp *serotinum, Ledum groenlandicum* and *Ravensara aromatica,* for simultaneous, separate or sequential use, for the treatment of cancerous or precancerous pathological conditions.

2. Association for use according to Claim 1,
**characterized in that** it consists in a composition comprising a mixture of the three said essential oils of *Helichrysum italicum* ssp *serotinum, Ledum groenlandicum* and *Ravensara aromatica.*

3. Association for use according to either of Claims 1 and 2, **characterized in that** it comprises at least 10% by weight, preferably at least 20% by weight, of each of said essential oils, relative to the total weight of said essential oils.

4. Pharmaceutical association for use according to one of Claims 1 to 3, **characterized in that** it comprises the following compounds:
- 1,8-cineol;
- at least one alcohol chosen from alpha-terpineol and 1-terpinen-4-ol;
- terpene compounds chosen from the compounds comprising sabinene, pinene, terpinene, limonene, phellandrene, terpinolene, selinene, curcumene, germacrene, cymene, caryophyllene, thujene and ocimene;
- esters comprising neryl acetate and/or neryl propionate, and
- ketone compounds comprising at least one sesquiterpene ketone, preferably germacrone, and at least one monoterpene ketone chosen from nonanone, carvone, undecanone and 2,5,7-trimethyl-2-decene-6,8-dione.

5. Association for use according to Claim 4,
**characterized in that** it comprises the following main compounds:
- 1,8-cineol;
- alpha-terpineol;
- neryl acetate;
- germacrone, and
- terpene compounds chosen from germacrene, sabinene, limonene, phellandrene and selinene.

6. Pharmaceutical association for use according to one of Claims 1 to 5, **characterized in that** it aims at treating a tumour.

7. Pharmaceutical association for use according to one of Claims 1 to 5, **characterized in that** it aims at improving the general condition of the patient and the tolerance to chemotherapy.

## Patentansprüche

1. Assoziation, die drei ätherische Öle umfaßt, die aus den ätherischen Ölen des Helichrysum italicum ssp. serotinum, Ledum groenlandicum und Ravensara aromatica bestehen, für eine gleichzeitige, getrennte oder zeitlich gestaffelte Verwendung, zur Behandlung von kanzerösen und präkanzerösen Pathologien.

2. Assoziation für eine Verwendung nach Anspruch 1, **dadurch gekennzeichnet, daß** sie aus einer Zusammensetzung besteht, welche die drei genannten ätherischen Öle des Helichrysum italicum ssp. serotinum, Ledum groenlandicum und Ravensara aromatica in Mischung umfaßt.

3. Assoziation für eine Verwendung nach einem der Ansprüche 1 oder 2, **dadurch gekennzeichnet, daß** sie wenigstens 10 Gew.-%, vorzugsweise wenigstens 20 Gew.-% eines jeden der ätherischen Öle bezogen auf das Gesamtgewicht der ätherischen Öle umfaßt.

4. Pharmazeutische Assoziation für eine Verwendung nach einem der Ansprüche 1 bis 3, **dadurch gekennzeichnet, daß** sie die folgenden Verbindungen umfaßt:
- 1,8-Cineol,
- wenigstens einen Alkohol, der aus alpha-Terpineol und Terpinen-1-ol-4 ausgewählt ist,
- Terpenverbindungen, die aus den Verbindungen umfassend Sabinen, Pinen, Terpinen, Limonen, Phellandren, Terpinolen, Selinen, Curcumen, Germacren, Cymen, Caryophyllen, Thujen, Ocimen ausgewählt sind,
- die Ester, welche Nerylacetat und/oder Nerylpropionat umfassen, sowie
- die Ketonverbindungen, welche wenigstens ein Sesquiterpen-Keton, vorzugsweise Germacron, sowie wenigstens ein Monoterpen-Keton, ausgewählt aus Nonanon, Carvon, Undecanon und 2,5,7-Trimethyl-dec-2-en-6,8-dion umfassen.

5. Assoziation für eine Verwendung nach Anspruch 4, **dadurch gekennzeichnet, daß** sie die folgenden Hauptverbindungen umfaßt:
- 1,8-Cineol,
- alpha-Terpineol,
- Nerylacetat,
- Germacron und
- Terpenverbindungen, ausgewählt aus Germacren, Sabinen, Limonen, Phellandren und Selinen.

6. Pharmazeutische Assoziation für eine Verwendung nach einem der Ansprüche 1 bis 5, **dadurch gekennzeichnet, daß** sie für die Behandlung eines Tumors bestimmt ist.

7. Pharmazeutische Assoziation für eine Verwendung nach einem der Ansprüche 1 bis 5, **dadurch gekennzeichnet, daß** sie dafür bestimmt ist, den Allgemeinzustand des Patienten zu verbessern und die Chemotherapietoleranz zu erhöhen.
